# EUROPEAN PATENT SPECIFICATION

(11) **EP 2 016 936 B1**
(45) Date of publication and mention of the grant of the patent: **15.09.2010**
(21) Application number: 07112104.0
(22) Date of filing: 09.07.2007
(51) Int. Cl.: A61K 9/00, A61K 9/107, A61K 31/196

(54) **Oil in water emulsion comprising NSAIDs and quaternary ammonium halides**
Öl in Wasser Emulsionen enthaltend NSAID und quartär Ammoniumhalogenide
Émulsion huile dans eau comprenant AINS et halogénure d'ammonium quaternaire

(43) Date of publication of application: 21.01.2009
(73) Proprietor: Novagali Pharma S.A., 91000 Evry (FR)
(72) Inventor: Lambert, Grégory, 92290, CHATENAY MALABRY (FR); Rabinovich-Guilatt, Laura, 60920, KADIMA (IL); Lallemand, Frédéric, 94260, FRESNES (FR); Garrigue, Jean-Sébastien, 91370, VERRIERE LE BUISSON (FR)
(74) Representative: Icosa

(56) References cited:
- EP-A- 0 390 071
- EP-A- 0 696 452
- EP-A- 1 688 144
- WO-A-2007/042262
- US-A- 5 110 493
- ABDULRAZIK M ET AL: "OCULAR DELIVERY OF CYCLOSPORIN A II. EFFECT OF SUBMICRON EMULSION'S SURFACE CHARGE ON OCULAR DISTRIBUTION OF TOPICAL CYCLOSPORIN A" SCIENCES TECHNIQUES ET PRATIQUES STP PHARMA SCIENCES, PARIS, FR, vol. 11, no. 6, 2001, pages 427-432, XP008033036 ISSN: 1157-1489

## Description

### [Field of the invention]

This invention relates to pharmaceutical, including ophthalmic compositions comprising non-steroidal anti-inflammatory drugs (NSAIDs), more preferably to ophthalmic emulsions being useful for the treatment of eye conditions. This invention also relates to oil-in-water emulsions comprising NSAIDs and further including at least one quaternary ammonium compound as cationic agent.

### [Background]

The topical use of NSAIDs in the treatment of ophthalmic diseases was first taught in US 4,454,151. This US patent disclosed efficacious formulations with NaH2PO4H2O, Na2PO4H2O, NaCl, benzalkonium chloride (BAK) and sterilized water. However, these formulations were found to not have the stability required for shelf life in formulations.

Ocufen Ophthalmic solution, a non-patented medicament launched in 1988, is the first NSAID (flurbiprofen) approved by the FDA for ophthalmic use, and incorporates thimerosal as its preservative system. Thimerosal is an irritating organomercury compound (approximately 49% mercury by weight), and is known for its teratogenic side effects. New generations of ophthalmic formulations deprived from thimerosal are therefore needed.

US patent 5,110,493 disclosed improved and stable ophthalmic solutions (but no emulsions) including NSAID and using BAK as preservative. This formulation comprises 0.001% to 10% of NSAID, 0.001% to 1% of preservative such as BAK, 0.001% to 1% of surfactant such as octoxynol 40, excipients and water. This formulation was shown to be stable for at least the minimum reasonable shelf life of such products. However, bioavailability is poor: typical dosage ranges for this formulation to treat an eye condition is disclosed to be about 2-10 drops of 0.1% solution of NSAID per day.

This raises the problem of bioavailability of topically instilled drugs such as NSAIDs. Most of these NSAIDs exhibit complex ocular formulation problems due to aqueous solubility limitations. Therefore, attempts have been made to improve ocular bioavailability of NSAIDs by designing new colloidal delivery systems based either on nanoparticles, and negatively or positively charged submicron emulsions. Examples of such emulsions are disclosed in Klang et al (Journal of Controlled Release, 1999, 57:19-27): NSAID 0.1%, MCT 8.5%, Lipoid E80 1.2%, stearylamine 0.3%, alpha-tocopherol 0.02%, poloxamer 188 1%, glycerol 2.25% and water. The Applicant worked on different ophthalmic emulsions for NSAIDs in order to obtain an emulsion having a better stability and providing a better ocular bioavailability of NSAIDs.

Therefore, the Applicant worked on long chain quaternary ammonium compounds such as BAK, and noticed that the length of the alkyl chain was important with regards to the function performed by the quaternary ammonium compounds in an oil-in-water emulsion containing NSAIDs: acting on the length of the alkyl chain resulted in enhancing or reducing the cationic power of the quaternary ammonium compounds. Without wanting to be linked by any theory, the Applicant observed on working on oil-in-water emulsions containing NSAIDs, that long chain alkyl quaternary ammonium compounds were preferentially localized at the oil/water interface of the emulsions, resulting in (1) emulsions with higher zeta potential (2) more stable emulsions.

In addition, the Applicant observed that an emulsion comprising NSAID and a quaternary ammonium halide in which the nitrogen atom is substituted by one or more alkyl group having at least 12, preferably 14 or 16, more preferably 16 carbon atoms provides a better ocular bioavailability compared to others formulations, which is of importance and resulted in the design of therapeutic emulsions having a content in oil of less than 6%, preferably of 5% or less, thus less irritating than the prior art emulsion. Therefore, the goal of this invention is to provide an oil-in-water emulsion comprising a non-steroidal anti-inflammatory drug and a quaternary ammonium halide in which the nitrogen atom is substituted by one or more alkyl group having at least 12 carbon atoms, preferably 14 or 16 carbon atoms.

According to the invention, said emulsion is useful for ophthalmic purposes.

### [Description of the invention]

In the meaning of this invention,
"Cationic emulsions" are emulsions having a positive zeta potential, preferably a zeta potential higher to 10 mV;
"long chain alkyl" are alkyl moieties having at least 12 carbon atoms, preferably at least 14 carbon atoms, more preferably at least 16 carbon atom;
n-alkyl dimethyl benzyl ammonium chloride also called benzalkonium chloride (hereinafter also referred to as BAK or BAC)
"BAK C12" refers to benzododecinium chloride (CAS 139-07-1);
"BAK C14" refers to myristalkonium chloride (CAS 139-08-2);
"BAK C16" refers to cetalkonium chloride (CAS 122-18-9);
"MCT" means Medium chain triglycerides; for the experimentation, TCM^{™} (Societe des Oleagineux, France) was the MCT used.

"Flurbiprofen" means a compound selected from 2-((3-fluoro-4-phenyl)phenyl)propanoic acid and salts thereof, preferably the sodium salt, and hydrates thereof and flurbiprofen anhydride ; the term " flurbiprofen" in the meaning of this invention also includes flurbiprofen derivatives, especially flurbiprofen acid derivatives wherein the acid function is protected by any suitable protecting group, or flurbiprofen amide derivatives. The term " flurbiprofen" in the meaning of this invention may designate (1) racemic flurbiprofen, i.e. a mixture of (S)-flurbiprofen and (R)-flurbiprofen, and includes not only a mixture of (S)-flurbiprofen and (R)-flurbiprofen at a molar ratio of 50:50, but also a mixture of (S)-flurbiprofen and (R)-flurbiprofen at a molar ratio from 20:80 to 80:20, preferably from 30:70 to 70:30, (2) or pure or enriched enantiomer forms, such as for example (R)-flurbiprofen or S-flurbiprofen, preferably S-flurbiprofen as well as pharmaceutical acceptable salts.

This invention thus relates to an ophtalmic oil-in-water emulsion comprising at least one NSAID and at least one quaternary ammonium halide, more preferably ammonium chloride or bromide, in which the nitrogen atom of the ammonium group is substituted by at least one or only one alkyl group having at least 12 carbon atoms, more preferably by one alkyl group having at least 14 carbon atoms, more preferably by one alkyl group having at least 16 carbon atoms.

According to an embodiment, the non-steroidal anti-inflammatory drug is chosen among ketorolac, salicylate, indomethacin, ibuprofen, diclofenac, flurbiprofen, suprofen, piroxicam, COX2 inhibitors, diclofenac, nimesulide, nepafenac, including any derivatives, prodrugs and salts thereof.

Advantageously, said non-steroidal anti-inflammatory drug is flurbiprofen.

According to an embodiment, the therapeutic oil-in-water emulsion of the invention comprises 0.001% to 10% preferably 0.01% to 1%, more preferably 0.02% to 0.05% of a non-steroidal anti-inflammatory drug, preferably flurbiprofen, in weight by total weight of the emulsion.

According to an embodiment, the quaternary ammonium halide is a C16-alkyl quaternary ammonium halide, preferably C16-alkyl benzyl dimethyl ammonium chloride.

Advantageously, the ammonium halide is BAK C12, BAK C14, BAK C16 or a mixture thereof. According to an embodiment, the only ammonium halide of the emulsion is BAK C14 or BAK C16 or a mixture thereof.

According to an embodiment, the therapeutic oil-in-water emulsion of the invention is cationic. By cationic oil-in water emulsion is understood an oil-in-water emulsion having a positive zeta potential. In this embodiment, the emulsion of the invention has a positive zeta potential. Advantageously, the emulsion of the invention keeps a positive zeta potential overtime.

According to an embodiment, the therapeutic oil-in-water emulsion of the invention comprises 0.001 to 0.1%, preferably 0.002 to 0.05%, more preferably 0.002 to 0.005% in weight of ammonium halide by total weight of the emulsion.

The therapeutic oil-in-water emulsion according to the invention further comprises an oil phase preferably comprising MCT, castor oil, soybean oil or any suitable vegetal or mineral oil, surfactants preferably chosen among at least one of tyloxapol, poloxamer, tocopherol, polyethylene glycol succinate and polysorbate, sorbitan monolaurate, and optionally antioxidants and/or isotonicity agents preferably chosen among at least one of glycerol, NaCl and mannitol. According to an embodiment, the therapeutic oil-in-water emulsion of the invention comprises 0.1 to 5 %, preferably 0.5 to 4 %, more preferably 1 to 3% in weight of oil, preferably MCT, by total weight of the emulsion. According to an embodiment, the therapeutic oil-in-water emulsion of the invention comprises 0.01 to 2 %, preferably 0.05 to 1 %, more preferably 0.1 to 0.5% in weight of surfactants to the total weight of the emulsion. According to an embodiment, the emulsion of the invention includes at least one surfactant in the oily phase, preferably in an amount of 0.1 to 0.5 %, preferably 0.3% in weight to the total weight of the emulsion and at least one surfactant in the aqueous phase preferably in an amount of 0.02 to 0.2 %, preferably 0.1% in weight to the total weight of the emulsion. Preferred surfactants are tyloxapol, poloxamer and sorbitan monolaurate.

According to an embodiment, the emulsion of the invention includes buffering agents such as citrate, phosphate, tris, borate, acetate, carbonate, borate-polyol complexes, histidine, gluconate or lactate buffers.

According to a an embodiment, the pH of the emulsion is chosen in order to optimize the incorporation and solubilisation of the NSAID into the oil phase.

According to an embodiment, the therapeutic oil-in-water emulsion of the invention has a droplet size of 100 to 500 nm, preferably 150 to 400 nm, more preferably 110 to 250 nm.

According to an embodiment, the therapeutic oil-in-water emulsion of the invention is preserved.

According to another embodiment, the oil-in-water emulsion of the invention is unpreserved.

According to an embodiment, the therapeutic oil-in-water emulsion of the invention is presented in single use units. According to another embodiment, the oil-in-water emulsion of the invention is marketed in multidose containers.

This invention also relates to a medicament comprising a therapeutic oil-in-water emulsion of the invention.

This invention also relates to the use of an oil-in-water emulsion according to the invention for the manufacture of a medicament for the treatment of an eye disease caused by, associated with or accompanied by inflammatory processes. In the meaning of the invention, eye diseases caused by, associated with or accompanied by inflammatory processes means a wide variety of ocular conditions such as for example glaucoma, ocular inflammatory conditions such as keratitis, uveitis, intra-ocular inflammation, allergy and dry-eye syndrome ocular infections, ocular allergies, ocular infections, retinal edema, macular edema, diabetic retinopathy, or any trauma caused by eye surgery or eye injury, LASIK surgery, Periperi surgery (pre, per and/or post) for prevention and treatment of pain and inflammation.

This invention also relates to the combination of an NSAID further comprising other active drugs for the treatment of eye diseases, where both compound are within an oil-in-water emulsion according to the invention. According to an embodiment, said other active drug is an immunosuppressive agent, preferably cyclosporine, sirolimus or tacrolimus. According to an embodiment, in the emulsion of the invention, NSAID is combined with cyclosporin A. In a preferred embodiment, this combination shows synergistic effects. By synergistic effects, it is understood that the combination has better efficacy than the separate administration of the two molecules, or that it allows reducing the dose or administration frequency compared to the two separate entities.

Advantageously, the medicament is administrated less than 4 times a day, preferably less than three times a day and more preferably once daily. According to an embodiment, the medicament may be both curative and preventive. Where applied, for example, pre-surgically or immediately post-traumatically, i.e. before inflammation develops, the emulsion of the invention prevents development of inflammation. When applied directly to the eye suffering from any of the named ophthalmic diseases, it suppresses or attenuates or limits already existing inflammatory processes. This invention thus relates to the use of the therapeutic oil-in-water emulsion or of a medicament containing such, for preventing inflammation, and/or for treating inflammation when applied to an eye.

This invention also relates to a therapeutic ophthalmic oil-in-water emulsion or a medicament containing such, in a unitary dosage form, for a single use. Advantageously, in this embodiment, the emulsion is sterile.

Another object of this invention is a pre-concentrate of the therapeutic oil-in-water emulsion of the invention and a process for manufacturing said pre-concentrate.

According to this invention, a pre-concentrate is defined as an emulsion having an amount of oil higher than the amount of oil of the therapeutic emulsion administered to a patient. The amount of oil in the pre-concentrate is of at least 20 % v/v, more preferably of at least 30% v/v.

The pre-concentrate may be in a liquid form or in a gel form, or in any form suitable in view of its further dilution with water.

According to an embodiment, the pre-concentrate of ophthalmic oil-in-water emulsion according to the present invention may be sterilized, for example, by heat, such as by autoclaving, or by filtering or filtration, or by irradiation, or by gas sterilization. In another embodiment, the concentrate of the ophthalmic emulsion is prepared in an aseptic manner.

This invention also relates to a process for manufacturing a pre-concentrate of a therapeutic oil-in-water emulsion comprising the steps of emulsifying/mixing the oil phase with an aqueous phase and with surface-active component(s), wherein the non-steroidal anti-inflammatory drug is dissolved in the oil phase. The process for manufacturing said pre-concentrate comprises emulsifying an amount of oil with an aqueous phase and with suitable surfactants, in order to obtain an emulsion having an amount in oil higher than the amount in oil of the corresponding emulsion to be administered for therapeutic purposes.

Before beginning the manufacturing process, the therapeutic oil-in-water emulsion is designed, with a wished concentration of oil, the type of oil (suitable for ophthalmic use, such as for example castor oil, MCT ...), the type of elements needed for emulsification such as surfactants for example, and one or more NSAID. The concentration of the concentrate is then decided, depending on the industrial volumes needed.

This invention also relates to a process for manufacturing a therapeutic oil-in-water emulsion comprising (1) manufacturing a pre-concentrate of an ophthalmic oil in water emulsion, said pre-concentrate having a content in oil of at least 4 % v/v, preferably of 10% v/v or more, more preferably of 20% v/v or more, even more preferably of 30% v/v or more by emulsifying/mixing an oil suitable for ophthalmic use selected in the group comprising mineral oil, castor oil and MCT, said oil phase containing one or more NSAID and preferably containing flurbiprofen, with an aqueous phase comprising a quaternary ammonium halide and with surface-active component(s) and then(2) diluting one volume of the resulting pre-concentrate with 2 to 50 volumes of water.

According to an embodiment, the emulsification is such that the droplet size or the distribution of the droplet size in the pre-concentrate is about the same as the droplet size or the distribution of the droplet size of the therapeutic oil-in-water emulsion.

According to an embodiment, the diluting water may comprise additives selected from the group comprising tonicity agents, such as for example NaCl, glycerol or mannitol, viscosifying agents, buffering agents, preservatives, antioxidants or colorants.

According to an embodiment, the diluting water may also comprise a quaternary ammonium halide.

Then, according to the invention, a pre-concentrate of this desired emulsion is produced by mixing the oil suitable for ophthalmic use, with an aqueous phase and with surface-active component(s); the average hydrophilic-lipophilic balance (HLB) of the surface-active component(s) may advantageously be about equal to the HLB or average HLB emulsion requirement of the oil or oils used in the present compositions.

An advantage of this invention is to produce large volumes of emulsions without having to scale-up the emulsifying process.

This invention relates to a process for manufacturing a therapeutic oil-in-water emulsion according to the invention, comprising manufacturing a concentrate according to the above-mentioned process and then diluting said concentrate, by mixing 1 volume of concentrate with 2 to 50 volumes of water, to obtain a final therapeutic emulsion having an oil content of 5% v/v of less, preferably of 3% v/v or less, more preferably of 2% v/v or less, even more preferably of 1% v/v or less.

This invention also relates to a method for the treatment of ocular diseases or conditions consisting in the administration to a patient of an ophthalmic emulsion prepared from a pre-concentrate, according to the above described process.

The invention also relates to oil-in-water emulsions obtainable by the process of the invention, i.e. by manufacturing a concentrate including at least one NSAID such as for example flurbiprofen, and then diluting said concentrate with 2 to 50 volumes of water, said water optionally comprising additives, such as for example tonicity agents, viscosifying agents, buffering agents, preservatives, antioxidants or colorants.

One advantage of the invention is that the oil-in-water emulsions obtained by dilution of the concentrates are formed with reduced energy input.

The following examples and figures illustrate the invention and should not be interpreted in any way as reducing the scope of this invention.

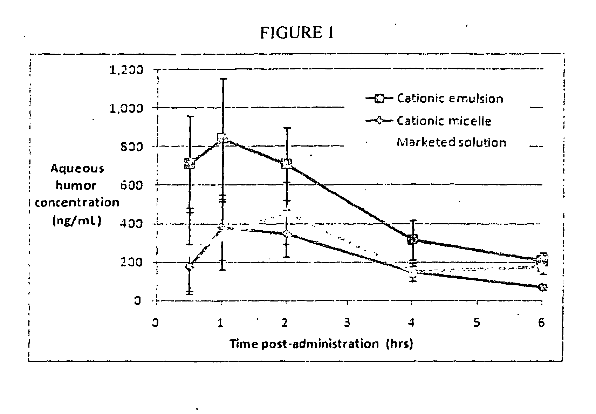

Fig. 1 refers is a graph showing bioavailability of the emulsions of the invention. This graph shows the aqueous humour concentration of flurbiprofen overtime after administration.

Example of formulations of an oil-in-water emulsion:

**Emulsion 1 (Table 1):**

| **PHASE** | **INGREDIENT** | **FUNCTION** | **CONC CIBLE% w/w** |
|---|---|---|---|
| **Oily phase** | Flurbiprofen anhydride | Active Ingredient | 0.048% |
| | Medium-Chain Triglycerides | Oily agent | 4.00% |
| | Tyloxapol | Surfactant | 0.30% |
| **Aqueous phase** | BAK C16 (cetalkonium chloride) | Cationic surfactant | 0.005% |
| | Sorbitane monolaurate | Surfactant | 0.1% |
| | Glycerin | Tonicity agent | 2.5% |
| | Water | Diluent | 95.23 |

**Emulsion 2 (Table 2):**

| **PHASE** | **INGREDIENT** | **FUNCTION** | **CONC CIBLE% w/w** |
|---|---|---|---|
| **Oily phrase** | Flurbiprofen anhydride | Active Ingredient | 0.048% |
| | Medium-Chain Triglycerides | Oily agent | 4.00% |
| | Tyloxapol | Surfactant | 0.30% |
| **Aqueous phase** | BAK C16 (cetalkonium chloride) | Cationic surfactant | 0,005% |
| | Poloxamer 188 | Surfactant | 0.1% |
| | Glycerin | Tonicity agent | 2.5% |
| | Water | Diluent | 95.23 |

**Emulsion 3 (Table 3):**

| **PHASE** | **INGREDIENT** | **FUNCTION** | **CONC CIBLE% w/w** |
|---|---|---|---|
| **Oily phase** | Flurbiprofen anhydride | Active Ingredient | 0.03% |
| | Medium-Chain Triglycerides | Oily agent | 2.00% |
| | Tyloxapol | Surfactant | 0.30% |
| **Aqueous phase** | BAK C16 (cetalkonium chloride) | Cationic surfactant | 0.005% |
| | Poloxamer 188 | Surfactant | 0.1% |
| | Glycerin | Tonicity agent | 2.5% |
| | Water | Diluent | 95.23 |

**Stability of the Emulsion 2(Table 4):**

| | **T0** | **7days at 80°C** | **14days at 80°C** |
|---|---|---|---|
| Zeta potential (mV) | 27.6 | 24.5 | 22.6 |
| pH | 5,41 | 4,44 | 4,41 |
| Mean droplet size (nm) | 187 | 185 | 202 |
| Osmolality (mOsm/kg) | 313 | 321 | 322 |
| Flurbiprofen concentration (% of theoretical) | 100% | Stable | Stable |

### In vivo study:

Forty-five (45) pigmented rabbits were divided into three groups of fifteen animals corresponding to three treatments with five time-points each (0.5, 1, 2, 4 and 6 hours after administration). All animals were treated with a 50 µl single instillation in each eye of 50 µl of flurbiprofen at 0.03% (Emulsion, cationic micelle and marketed solution).

At the respective time-points aqueous humor was sampled from both eyes for flurbiprofen determination.

For this invivo study, the emulsion and the micelle are as described in Table 4 and Table 5. Ocufen® is an ophthalmic solution containing excipients such as buffer citrate (pH 6.45); edetate disodium; polyvinyl alcohol (1.4%); potassium chloride; purified water and sodium chloride. It has a pH of 6.0 to 7.0 and an osmolality of 260 - 330 mOsm/kg. The multidose version contains 0.005% thimerosal as preservative.

**Table 4: emulsion of the invention for invivo study**

| | | **Emulsion Theoretical content (% w/w)** |
|---|---|---|
| **Oily phase** | Flurbiprofen | 0.03 |
| | MCT | 2.00 |
| | Tyloxapol | 0.10 |
| | Sorbitan monolaurate | 0.05 |
| **Aqueous phase** | CKC (BAK C16) | 0.005 |
| | Glycerol | 2.50 |
| | Deionised water | qs 100 |

**Table 5: cationic micelles**

| | **Cationic micelles** **Theoretical content (% w/w)** |
|---|---|
| Flurbiprofen | 0.03 |
| Cremophor RH40 | 0.10 |
| CKC (BAK C16) | 0.005 |
| Glycerol | 2.50 |
| Deionised water | qs 100 |

Results shown in figure 1 evidence that cationic emulsions of the invention improves the ocular bioavailability compared to marketed solution and compared to cationic micelles.

## Claims

1. An ophthalmic oil-in-water emulsion comprising a non-steroidal anti-inflammatory drug and a quaternary ammonium halide in which the nitrogen atom is substituted by one or more alkyl group having at least 12 carbon atoms.

2. An oil-in-water emulsion according to claim **1,** wherein said non-steroidal anti-inflammatory drug is chosen among ketorolac, salicylate, indomethacin, ibuprofen, diclofenac, flurbiprofen, suprofen, piroxicam, COX2 inhibitors, diclofenac, nimesulide, nepafenac.

3. An oil-in-water emulsion according to claim **1,** wherein said non-steroidal anti-inflammatory drug is flurbiprofen.

4. An oil-in-water emulsion according to anyone of claims **1** to **3,** comprising 0.001% to 10% of a non-steroidal anti-inflammatory drug, preferably 0.01% to 1%, more preferably 0.02% to 0.05% w/w of the emulsion.

5. An oil-in-water emulsion according to anyone of claims **1** to **4,** wherein said quaternary ammonium halide is a C16-alkyl quaternary ammonium halide.

6. an oil-in-water emulsion according to anyone of claims **1** to **4,** wherein said quaternary ammonium halide is benzyl dimethyl ammonium chloride or bromide, in which the nitrogen atom is substituted by an alkyl group having at least 12 carbon atoms.

7. An oil-in-water emulsion according to claim **6,** wherein said quaternary ammonium halide is C16-alkyl benzyl dimethyl ammonium chloride.

8. An oil-in-water emulsion according to anyone of claims **1** to **4,** wherein said quaternary ammonium halide is a trimethyl ammonium chloride or bromide, wherein the nitrogen atom is further substituted by an alkyl group having at least 12 carbon atoms.

9. An oil-in-water emulsion according to anyone of claims **1** to **8,** further comprising an oil phase comprising MCT, castor oil or mineral oil, surfactants preferably chosen among at least one of tyloxapol, poloxamer, tocopherol, polyethylene glycol succinate, sorbitan monolaurate and polysorbate, and optionally antioxidants and/or isotonicity agents preferably chosen among at least one of glycerol and mannitol.

10. An oil-in-water emulsion according to anyone of claims **1** to **9,** comprising 0.001 to 0.1% of ammonium halide.

11. An oil-in-water emulsion according to anyone of claims **1** to **10,** said emulsion having a positive zeta potential.

12. An oil-in-water emulsion according to anyone of claims **1** to **11,** said emulsion having a droplet size of 100 to 500 nm.

13. An oil-in-water emulsion according to anyone of claims **1** to **12,** said emulsion being preserved.

14. An oil-in-water emulsion according to anyone of claims **1** to **12,** said emulsion being unpreserved.

15. An oil-in-water emulsion according to anyone of claims **1** to **14,** further comprising an immunosuppressive agent, preferably cyclosporin.

16. Medicament comprising an oil-in-water emulsion according to anyone of claims **1** to **15.**

17. Medicament according to claim **16,** in a unitary dosage form.

18. Medicament according to claim **16** or **17,** wherein the emulsion is sterile.

19. Use of an oil-in-water emulsion according to anyone of claims **1** to **15** for the manufacture of a medicament for the treatment of an eye disease caused by, associated with or accompanied by inflammatory processes.

20. Use of an oil-in-water emulsion according to anyone of claims **1** to **15** for the manufacture of a medicament to be applied pre-surgically or immediately post-traumatically in order to prevent inflammation or to prevent the development of an inflammatory process.

21. Use of an oil-in-water emulsion according to anyone of claims **1** to **15** for the manufacture of a medicament for the treatment of an inflammation or the limitation of an inflammatory process.

22. The use according to any of claims **19** to **21,** wherein said medicament is administrated less than 4 times a day, preferably less than twice a day and more preferably less than once a day.

23. Process for manufacturing a pre-concentrate of an oil in water emulsion according to anyone of claims **1** to **15,** said pre-concentrate having a content in oil of 20% v/v or more, the process comprising the steps of emulsifying/mixing the oil phase with an aqueous phase and with surface-active component(s), the non-steroidal anti-inflammatory drug being dissolved in the oil phase.

24. Pre-concentrate of a therapeutic oil-in-water emulsion, having a content in oil of 20% v/v or more, comprising a non-steroidal anti-inflammatory drug and a quaternary ammonium halide in which the nitrogen atom is substituted by one or more alkyl group having at least 12 carbon atoms, said pre-concentrate being obtainable by emulsifying/mixing the oil phase with an aqueous phase and with surface-active component(s), the non-steroidal anti-inflammatory drug being dissolved in the oil phase.

25. A process for manufacturing an ophthalmic oil in water emulsion according to anyone of claims **1** to **15,** comprising
(1) manufacturing a pre-concentrate of an ophthalmic oil in water emulsion, said pre-concentrate having a content in oil of at least 3 % v/v, preferably of 10% v/v or more, more preferably of 20% v/v or more, even more preferably of 30% v/v or more comprising the steps of emulsifying/mixing an oil suitable for ophthalmic use selected in the group comprising mineral oil, castor oil and MCT, with an aqueous phase and with surface-active component(s) and
(2) diluting a volume of the resulting pre-concentrate with 2 to 50 volumes of water, optionally containing a quaternary ammonium chloride, preferably BAK C16.

## Patentansprüche

1. Augen-Öl-in-Wasser-Emulsion, umfassend einen nicht steroidalen anti-inflammatorischen Wirkstoff und ein quartäres Ammoniumhalogenid, in dem das Stickstoffatom durch eine oder mehrere Alkylgruppen mit mindestens 12 Kohlenstoffatomen substituiert ist.

2. Öl-in-Wasser-Emulsion gemäß Anspruch 1, wobei der nicht steroidale anti-inflammatorische Wirkstoff ausgewählt ist aus Ketorolac, Salicylat, Indomethacin, Ibuprofen, Diclofenac, Flurbiprofen, Suprofen, Piroxicam, COX2-Inhibitoren, Diclofenac, Nimesulid, Nepafenac.

3. Öl-in-Wasser-Emulsion gemäß Anspruch 1, wobei der nicht steroidale anti-inflammatorische Wirkstoff Flurbiprofen ist.

4. Öl-in-Wasser-Emulsion gemäß einem beliebigen der Ansprüche 1 bis 3, umfassend 0,001 % bis 10 % eines nicht steroidalen anti-inflammatorischen Wirkstoffs, vorzugsweise 0,01 % bis 1 %, noch bevorzugter 0,02 % bis 0,05 % Gewichtsprozent von der Emulsion.

5. Öl-in-Wasser-Emulsion gemäß einem beliebigen der Ansprüche 1 bis 4, wobei das quartäre Ammoniumhalogenid ein C16-Alkyl-quartäres-Ammoniumhalogenid ist.

6. Öl-in-Wasser-Emulsion gemäß einem beliebigen der Ansprüche 1 bis 4, wobei das quartäre Ammoniumhalogenid Benzyl-dimethyl-Ammoniumchlorid oder -bromid ist, in dem das Stickstoffatom durch eine Alkylgruppe mit mindestens 12 Kohlenstoffatomen substituiert ist.

7. Öl-in-Wasser-Emulsion gemäß Anspruch 6, wobei das quartäre Ammoniumhalogenid C16-Alkyl-benzyl-dimethyl-Ammoniumchlorid ist.

8. Öl-in-Wasser-Emulsion gemäß einem beliebigen der Ansprüche 1 bis 4, wobei das quartäre Ammoniumhalogenid ein Trimethyl-Ammoniumchlorid oder -bromid ist, wobei das Stickstoffatom ferner durch eine Alkylgruppe mit mindestens 12 Kohlenstoffatomen substituiert ist.

9. Öl-in-Wasser-Emulsion gemäß einem beliebigen der Ansprüche 1 bis 8, des Weiteren umfassend eine Ölphase umfassend MCT, Rizinusöl oder Mineralöl, Tenside, vorzugsweise ausgewählt aus mindestens einem von Tyloxapol, Poloxamer, Tocopherol, Polyethylenglycol-Succinat, Sorbitanmonolaurat und Polysorbat, und wahlweise Antioxidantien und/oder isotonische Mittel, vorzugsweise ausgewählt aus mindestens einem von Glycerin und Mannit.

10. Öl-in-Wasser-Emulsion gemäß einem beliebigen der Ansprüche 1 bis 9, umfassend 0,001 bis 0,1% Ammoniumhalogenit.

11. Öl-in-Wasser-Emulsion gemäß einem beliebigen der Ansprüche 1 bis 10, wobei die Emulsion ein positives Zetapotential hat.

12. Öl-in-Wasser-Emulsion gemäß einem beliebigen der Ansprüche 1 bis 11, wobei die Emulsion eine Tropfengröße von 100 bis 500 nm hat.

13. Öl-in-Wasser-Emulsion gemäß einem beliebigen der Ansprüche 1 bis 12, wobei die Emulsion konserviert ist.

14. Öl-in-Wasser-Emulsion gemäß einem beliebigen der Ansprüche 1 bis 12, wobei die Emulsion unkonserviert ist.

15. Öl-in-Wasser-Emulsion gemäß einem beliebigen der Ansprüche 1 bis 14, des Weiteren umfassend ein immunsuppressives Mittel, vorzugsweise Cyclosporin.

16. Medikament umfassend eine Öl-in-Wasser-Emulsion gemäß einem beliebigen der Ansprüche 1 bis 15.

17. Medikament gemäß Anspruch 16 in einer einheitlichen Dosierungsform.

18. Medikament gemäß Anspruch 16 oder 17, wobei die Emulsion steril ist.

19. Verwendung einer Öl-in-Wasser-Emulsion gemäß einem beliebigen der Ansprüche 1 bis 15 für die Herstellung eines Medikaments für die Behandlung einer Augenkrankheit verursacht durch, assoziiert mit oder begleitet von Entzündungsprozessen.

20. Verwendung einer Öl-in-Wasser-Emulsion gemäß einem beliebigen der Ansprüche 1 bis 15 für die Herstellung eines Medikaments, welches vorchirurgisch oder unmittelbar post-traumatisch angewandt wird, um Entzündungen zu verhindern oder um die Entwicklung eines Entzündungsprozesses zu verhindern.

21. Verwendung einer Öl-in-Wasser-Emulsion gemäß einem beliebigen der Ansprüche 1 bis 15 für die Herstellung eines Medikaments für die Behandlung einer Entzündung oder der Eingrenzung eines Entzündungsprozesses.

22. Verwendung gemäß einem beliebigen der Ansprüche 19 bis 21, wobei das Medikament weniger als 4 Mal am Tag verabreicht wird, vorzugsweise weniger als zweimal am Tag und noch bevorzugter weniger als einmal am Tag.

23. Verfahren zum Herstellen eines Vorkonzentrats einer Öl-in-Wasser-Emulsion gemäß einem beliebigen der Ansprüche 1 bis 15, wobei das Vorkonzentrat einen Ölgehalt von 20 Vol.-% oder mehr hat, das Verfahren die Schritte des Emulgierens/Mischens der Ölphase mit einer wässrigen Phase und mit (einer) oberfächenaktiven Komponente(n) umfasst, wobei der nicht steroidale anti-inflammatorische Wirkstoff in der Ölphase gelöst ist.

24. Vorkonzentrat einer therapeutischen Öl-in-Wasser-Emulsion mit einem Ölgehalt von 20 Vol.-% oder mehr, umfassend einen nicht steroidalen anti-inflammatorischen Wirkstoff und ein quartäres Ammoniumhalogenit, in dem das Stickstoffatom durch eine oder mehrere Alkylgruppen mit mindestens 12 Kohlenstoffatomen substituiert ist, wobei das Vorkonzentrat durch Emulgieren/Mischen der Ölphase mit einer wässrigen Phase und mit (einer) oberfächenaktiven Komponente(n) erhältlich ist, wobei der nicht steroidale anti-inflammatorische Wirkstoff in der Ölphase gelöst ist.

25. Verfahren zum Herstellen einer Augen-Öl-in-Wasser-Emulsion gemäß einem beliebigen der Ansprüche 1 bis 15, umfassend
(1) Herstellen eines Vorkonzentrat einer Augen-Öl-in-Wasser-Emulsion, wobei das Vorkonzentrat einen Ölgehalt von mindestens 3 Vol.-%, vorzugsweise von 10 Vol.-% oder mehr, noch bevorzugter von 20 Vol.-% oder mehr, sogar noch bevorzugter von 30 Vol.-% oder mehr hat, umfassend die Schritte des Emulgierens/Mischens eines Öls, welches zur Augenanwendung geeignet und aus der Gruppe bestehend aus Mineralöl, Rizinusöl und MCT ausgewählt ist, mit einer wässrigen Phase und mit (einer) oberfächenaktiven Komponente(n) und
(2) Verdünnen eines Volumens des daraus resultierenden Vorkonzentrats mit 2 bis 50 Volumen an Wasser, wahlweise enthaltend ein quartäres Ammoniumchlorid, vorzugsweise BAK C16.

## Revendications

1. Émulsion ophtalmique du type huile dans l'eau comprenant un principe actif anti-inflammatoire non stéroïdien et un halogénure d'ammonium quaternaire dans lequel l'atome d'azote est substitué par un ou plusieurs groupes alkyles ayant au moins 12 atomes de carbone.

2. Émulsion du type huile dans l'eau selon la revendication **1,** dans laquelle ledit principe actif anti-inflammatoire non stéroïdien est choisi parmi le kétorolac, le salicylate, l'indométacine, l'ibuprofène, le diclofénac, le flurbiprofène, le suprofène, le piroxicam, les inhibiteurs de COX2, le diclofénac, le nimésulide, le népafénac.

3. Émulsion du type huile dans l'eau selon la revendication **1,** dans laquelle ledit principe actif anti-inflammatoire non stéroïdien est le flurbiprofène.

4. Émulsion du type huile dans l'eau selon l'une quelconque des revendications **1** à **3,** comprenant 0,001% à 10% d'un principe actif anti-inflammatoire non stéroïdien, préférablement de 0,01% à 1%, encore plus préférablement de 0,02% à 0,05% m/m de l'émulsion.

5. Émulsion du type huile dans l'eau selon l'une quelconque des revendications **1** à **4,** dans laquelle ledit halogénure d'ammonium quaternaire est un C16-alkyl halogénure d'ammonium quaternaire.

6. Émulsion du type huile dans l'eau selon l'une quelconque des revendications **1** à **4,** dans laquelle ledit halogénure d'ammonium quaternaire est le chlorure ou le bromure de benzyl diméthyl ammonium, dans lequel l'atome d'azote est substitué par un groupe alkyle ayant au moins 12 atomes de carbone.

7. Émulsion du type huile dans l'eau selon la revendication **6,** dans laquelle ledit halogénure d'ammonium quaternaire est le C16-alkyl benzyl diméthyl chlorure d'ammonium.

8. Émulsion du type huile dans l'eau selon l'une quelconque des revendications **1** à **4,** dans laquelle ledit halogénure d'ammonium quaternaire est le chlorure ou le bromure de triméthyl ammonium, dans lequel l'atome d'azote est de plus substitué par un groupe alkyle ayant au moins 12 atomes de carbone.

9. Émulsion du type huile dans l'eau selon l'une quelconque des revendications **1** à **8,** comprenant également une phase huileuse comprenant des TCM, de l'huile de ricin ou de l'huile de paraffine, des tensio-actifs préférablement choisis parmi au moins un tyloxapol, poloxamère, tocophérol, succinate de polyéthylène glycol, monolaurate de sorbitane et polysorbate, et éventuellement des antioxidants et/ou des agents d'isotonicité préférablement choisis parmi au moins le glycérol et le mannitol.

10. Émulsion du type huile dans l'eau selon l'une quelconque des revendications **1** à **9,** comprenant 0,001 à 0,1% d'halogénure d'ammonium.

11. Émulsion du type huile dans l'eau selon l'une quelconque des revendications **1** à **10,** ladite émulsion ayant un potentiel zêta positif.

12. Émulsion du type huile dans l'eau selon l'une quelconque des revendications **1** à **11,** ladite émulsion ayant une taille de gouttelette comprise entre 100 et 500 nm.

13. Émulsion du type huile dans l'eau selon l'une quelconque des revendications **1** à **12,** ladite émulsion étant conservée.

14. Émulsion du type huile dans l'eau selon l'une quelconque des revendications **1** à **12,** ladite émulsion n'étant pas conservée.

15. Émulsion du type huile dans l'eau selon l'une quelconque des revendications **1** à **14,** comprenant en outre un agent immunosuppresseur, de préférence la cyclosporine.

16. Médicament comprenant une émulsion du type huile dans l'eau selon l'une quelconque des revendications **1** à **15.**

17. Médicament selon la revendication **16,** dans un conditionnement unitaire.

18. Médicament selon les revendications **16** ou **17,** dans lequel l'émulsion est stérile.

19. Utilisation d'une émulsion du type huile dans l'eau selon l'une quelconque des revendications **1** à **15** pour la fabrication d'un médicament destiné au traitement d'une maladie oculaire due, associée ou accompagnée de processus inflammatoires.

20. Utilisation d'une émulsion du type huile dans l'eau selon l'une quelconque des revendications **1** à **15** pour la fabrication d'un médicament destiné à être appliqué avant une intervention chirurgicale ou immédiatement après un traumatisme afin de prévenir l'inflammation ou de prévenir le développement d'un processus inflammatoire.

21. Utilisation d'une émulsion du type huile dans l'eau selon l'une quelconque des revendications **1** à **15** pour la fabrication d'un médicament destiné au traitement d'une inflammation ou à la limitation d'un processus inflammatoire.

22. Utilisation selon l'une quelconque des revendications **19** à **21,** dans laquelle ledit médicament est administré moins de 4 fois par jour, préférablement moins de deux fois par jour et plus préférablement moins d'une fois par jour.

23. Procédé de fabrication d'un pré-concentré d'une émulsion huile dans l'eau selon l'une quelconque des revendications **1** à **15,** ledit pré-concentré ayant une teneur en huile de 20% v/v ou plus, encore plus préférablement de 30% v/v ou plus, le procédé comprenant les étapes d'émulsification/mélange de la phase huileuse avec une phase aqueuse et avec le(s)composé(s) tensio-actif(s), le principe actif anti-inflammatoire non stéroïdien étant dissout dans la phase huileuse.

24. Pré-concentré d'une émulsion thérapeutique huile dans l'eau, ayant une teneur en huile de 20% v/v ou plus, comprenant un principe actif anti-inflammatoire non stéroïdien et un halogénure d'ammonium quaternaire dans lequel l'atome d'azote est substitué par un ou plusieurs groupes alkyles ayant au moins 12 atomes de carbone, ledit pré-concentré étant susceptible d'être obtenu par émulsification/mélange de la phase huileuse avec une phase aqueuse et avec le(s)composé(s) tensio-actif(s), le principe actif anti-inflammatoire non stéroïdien étant dissout dans la phase huileuse.

25. Procédé pour la fabrication d'une émulsion ophtalmique huile dans l'eau selon l'une quelconque des revendications **1** à **15,** comprenant
(1) la fabrication d'un pré-concentré d'une émulsion ophtalmique huile dans l'eau, ledit pré-concentré ayant une teneur en huile d'au moins 3 % v/v, préférablement de 10% v/v ou plus, plus préférablement de 20% v/v ou plus, encore plus préférablement de 30% v/v ou plus comprenant les étapes d'émulsification/mélange d'une huile adaptée à une utilisation ophtalmique sélectionnée parmi le groupe comprenant une huile minérale, l'huile de ricin et le TCM, avec une phase aqueuse et avec le(s)composé(s) tensioactif(s) et
(2) la dilution d'un volume du pré-concentré résultant avec 2 à 50 volumes d'eau, contenant éventuellement un halogénure d'ammonium quaternaire, préférablement BAC C16.
